# EUROPEAN PATENT APPLICATION

(11) **EP 4 755 287 A1**
(43) Date of publication of application: **10.06.2026**
(21) Application number: 24307034.9
(22) Date of filing: 05.12.2024
(51) Int. Cl.: A61B 3/00, A61B 3/16, A61B 3/18

(54) **APPARATUS FOR DETERMINING A CHARACTERISTIC OF AN EYE**

(71) Applicant: Essilor International, 94220 Charenton-Le-Pont (FR)
(72) Inventor: ALAIMO, Salvatore, 94210 SAINT MAUR DES FOSSES (FR); HARDIN, Thomas, 94300 VINCENNES (FR); NAUCHE, Michel, 83240 CAVALAIRE-SUR-MER (FR)
(74) Representative: Jacobacci & Partners France

(57) **Abstract**

The disclosure relates to an apparatus (10) for determining a value of at least one characteristic of an eye of an individual (200), comprising:
- a housing (22),
- a individual's head support (25),
- a tonometer arm (100) configured to determine the value of the at least one characteristic, and movable relative to the housing between a position of use and a retracted position distinct from the position of use, and
wherein the housing comprises positioning means suitable to determine the position of the individual's eye relative to the housing when the individual's head rests on the individual's head support, whether the tonometer arm is in retracted position or in position of use.

## Description

### TECHNICAL FIELD OF THE DISCLOSURE

The present disclosure generally relates to ophthalmic measurement devices and more particularly to a tonometer designed to measure an intraocular pressure.

### BACKGROUND INFORMATION AND PRIOR ART

A tonometer is an instrument used to measure the pressure inside the eye of an individual, known as intraocular pressure (IOP). This measurement is crucial for diagnosing glaucoma, a condition characterized by increased pressure that can lead to optic nerve damage and vision loss if left untreated. There are several types of tonometers, including non-contact tonometers that measure the pressure based on the cornea's response to an air puff.

These non-contact tonometers are often preferred for the individual's comfort and for the accuracy of the given results.

Such a non-contact tonometer is designed to measure an intraocular pressure using an air pulse mechanism combined with a LED and a photodiode.

To this end, the tonometer emits a brief pulse of air (also called "puff of air") towards the cornea of an individual, causing it to deform. During the deformation, the corneal surface undergoes a sequence of shape changes: starting from its normal convex state, flattening, becoming concave, and then returning to its normal convex state. This process is monitored using the LED and the photodiode that determine the precise moment when the cornea is flat. The time taken for the cornea to reach this flat state is then used to calculate the eye intraocular pressure (this method is often referred to as the "air-puff test").

The air pulse must be brief and well centered on the cornea to ensure accurate and repeatable measurements. Any centering problem can affect the pressure point's consistency on the corneal surface, leading to erroneous readings.

That is why the tonometer usually comprises positioning means to ensure the air pulse to be well centered with the individual's eye.

Typically, such a tonometer can operate in several operating modes and the positioning means are designed to determine the position of the eye relative to the tonometer in one of these modes (just before the measurement of the intraocular pressure).

### SUMMARY OF THE DISCLOSURE

In this context, the present disclosure provides another architecture that ensures the positioning means can be used in several operating modes.

More precisely, the disclosure relates to an apparatus for determining a value of at least one characteristic of an eye of an individual, comprising:
- a housing,
- a individual's head support,
- a tonometer arm that is configured to determine the value of the at least one characteristic and that is movable relative to the housing between a position of use and a retracted position distinct from the position of use, and
wherein the housing comprises positioning means suitable to determine the position of the individual's eye relative to the housing when the individual's head rests on the individual's head support, whether the tonometer arm is in retracted position or in position of use.

Thus, thanks to the disclosure, regardless of the mode in which the tonometer is (and in particular regardless of the position of the arm), the positioning means are suitable for determining the position of the eye.

This feature has several advantages.

First of all, it allows an eye position measurement to be made at any time, which can be useful for making intraocular pressure measurements faster.

Furthermore, in the case the device is suitable to perform measurements of different characteristics of an eye, these only positioning means can be used before carrying out distinct kinds of measurements. This is an advantage relative to the prior art where, when the tonometer belonged to an assembly suitable for performing distinct measurements on an eye, each function comprised its own positioning means (which was expensive).

Other preferred features of the disclosure are the following ones:
- the apparatus comprises a determining unit fixed relative to the housing and suitable to determine a value of at least one other characteristic of the eye when the individual's head rests on the individual's head support.
- the apparatus comprises fluid supply means.
- the tonometer arm includes directing means suitable to eject the fluid coming from the fluid supply means toward the eye, along an outlet axis, when the individual's head rests on the individual's head support and when the tonometer arm is in the position of use.

- the directing means comprises:
   ¤ a plenum chamber body that has a side wall and two transparent end walls closing extremities of the side wall,
   ¤ an inlet pipe connected to a fluid inlet of the plenum chamber body and to the air supply means to supply the plenum chamber body with the fluid,
   ¤ an outlet pipe connected to a fluid outlet of the plenum chamber body and adapted to eject the fluid outside of the plenum chamber body along the outlet axis.
- the positioning means comprise:
   ¤ at least one light-emitting diode suitable to illuminate the individual's eye through the tonometer arm when this tonometer arm is in position of use, and
   ¤ at least one acquisition unit, such as a camera, suitable to acquire data relative to the position of the illuminated individual 's eye relative to the housing, through the tonometer arm when this tonometer arm is in position of use.
- the at least one light-emitting diode and the at least one acquisition unit are positioned such that a light ray emitted by the at least one light-emitting diode passes through the two transparent end walls of the plenum chamber body, is reflected by the individual's eye and comes back through the two transparent end walls until the at least one acquisition unit.
- the positioning means comprise at least three light-emitting diodes distributed along a circle centered on an outlet axis, and the acquisition unit is centered on the outlet axis.
- the housing includes a dome facing the individual 's head support, the apex of which is directed away from the individual's head support.
- the light-emitting diodes are located on the dome and the acquisition unit is located inside the housing, the apex of the dome being opened.
- the tonometer arm includes directing means for ejecting a fluid toward the eye of the individual along an outlet axis and wherein the tonometer arm is mounted sliding relative to the housing along a sliding axis tilted relative to the outlet axis.
- the individual's head support defines a support plane for an individual's forehead which is orthogonal to the outlet axis and wherein the sliding axis is tilted relative to the outlet axis such that the tonometer arm moves away from the support plane when it moves from its retracted position to its position of use.
- the sliding axis is inclined relative to the outlet axis by an angle lying between 70 and 89 degrees.
- the sliding of the tonometer arm is motorized.
- the value of the at least one characteristic is determined by measuring means, the measuring means comprising a lighting unit and a light sensor unit that are housed in the tonometer arm and oriented along axes intersecting each other.
- the housing comprises a visor that is adapted to house the tonometer arm in retracted position.
- the position of use is the position in which the at least one characteristic can be measured.
- the apparatus comprises a support on which the housing is mounted with at least one mobility.
- the individual's head support comprises a pedestal fixed relative to the support and a chin rest mounted on the pedestal with at least one mobility.

### DETAILED DESCRIPTION OF EXAMPLE(S)

The following description with reference to the accompanying drawings, given by way of non-limiting example makes it clear what the disclosure consists in and how it can be reduced to practice.

In the accompanying drawings:
- Figure 1 is a schematic perspective view of an apparatus according to the disclosure;
- Figure 2 shows two views of an individual's head resting on a support of the apparatus of Figure 1;
- Figure 3 is a side view of a front part of the housing of the apparatus of Figure 1;
- Figure 4 is a schematic perspective view of an arm of the apparatus of Figure 1;
- Figure 5 is a schematic sectional view in the plane A-A of Figure 4;
- Figure 6 is a schematic perspective view of a section of the arm of Figure 4;
- Figure 7 is a plan view of a plenum chamber body of the arm of Figure 4; and
- Figure 8 is a plan view of an eye of the individual and of the positioning means of the apparatus of Figure 1.

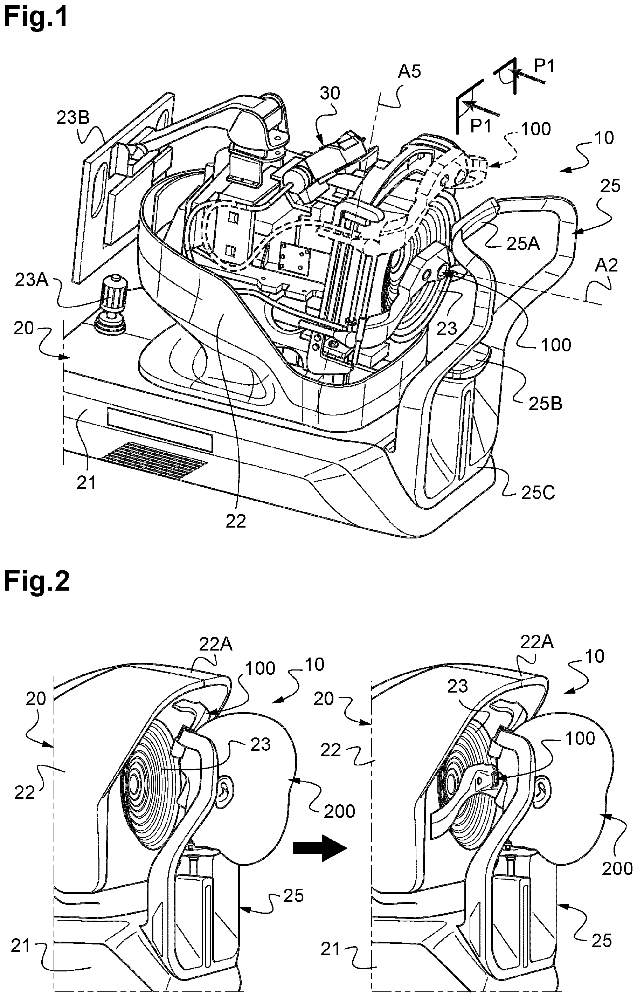

In Figure 1, an apparatus 10 having at least a tonometer function is shown.

This apparatus 10 comprises a base 20 and an arm 100 mounted on the base 20.

The base 20 here comprises a lower part (named base support 21) which is intended to be placed or fixed on a table and which is surmounted by an upper part (named housing 22).

The base support 21 and the housing 22 each include a protective cover which houses electrical, electronic and computer components.

In a preferred embodiment, the housing 22 is mounted on the base support 21 with at least one mobility. Here it is mounted on the latter with three mobilities. Specifically, the housing 22 can slide relative to the base support 21 along a longitudinal direction X, along a transverse direction Y and along a vertical direction Z (these directions being orthogonal to each other).

The base 20 further comprises a human-machine interface which, in the illustrated embodiment, comprises a joystick 23A mounted on the base support 21 and a display screen 23B mounted on the housing 22.

The base 20 also includes a individual's head support 25.

This support 25 allows an individual 200 to position his head in a satisfactory position and with a satisfactory orientation to carry out the required measurements (see Figure 2).

As shown in Figure 1, this support 25 here comprises a pedestal 25C that is fixed on the base support 21 and that has a frame shape. Its upper side includes a frontal rest 25A on which the individual can rest his forehead. This frontal rest 25A is here fixed relative to the base support 21. Its lower side includes a chin rest 25B on which the individual can rest his chin. This chin rest 25B is mounted on the pedestal 25C with a sliding mobility along a vertical direction Z, making it possible to adjust the vertical position of the individual's head relative to the base support 21.

Here, we will not describe the aforementioned components further, in the sense that they are well known to those skilled in the art.

We can only specify that the apparatus 10 is suitable to determine a value of a characteristic of an eye 201 of the individual 200 the head of which rests on the support 25. This characteristic is preferably the intraocular pressure of the individual's eye 201.

In a preferred embodiment, the apparatus 10 is suitable to determine a value of another characteristic of the eye 201 of the individual (when his head still rests on the support 25).

In other words, the apparatus 10 illustrated in Figure 1 has a tonometer function.

The apparatus could be a tonometer if its sole function were the measurement of intraocular pressure.

But this apparatus 10 preferably has other functions.

Typically, it can have at least one of the following functions:
- a corneal topography function,
- an interferometer function to measure the length of the eye 201,
- an aberrometer function for determining potential aberrations of the eye,
- an eye dry measurement function.

Each of these functions is well known and will not be described in more detail.

Here, the apparatus 10 illustrated in Figure 1 includes all these functions.

This is the reason why, in Figure 1, the protective cover of the upper part 22 of the base 20 has a front face (turned towards the individual) in the shape of an inverted dome 23.

The apparatus 10 also comprises a control unit that comprises a processing unit, such as a CPU, a programmable logic device (DSP, FGPA...) or a controller, or any combination thereof. It also comprises a memory and various input and output interfaces.

Thanks to its input interfaces, the processing unit is suitable for receiving instructions from the operator (through the human machine interface).

Thanks to its output interfaces, the processing unit is suitable for controlling the above functions and to display information on the display screen 23B.

Thanks to its memory, the processing unit stores either a computer application, consisting of computer programs comprising instructions, the execution of which by the processor enables the processing unit to implement the above functions, or logic gates designed to perform these functions.

To perform its function of pulsed-air tonometer, the apparatus 10 comprises air supply means 30 suitable for supplying air toward the individual eye 201 (the eye located in front of the arm 100 of the apparatus 10).

These air supply means 30 include an air system, making it possible to generate an air pulse when it receives a corresponding instruction from the control unit.

The air pulse is conducted to the eye 201 through the arm 100 by means of "directing means".

This arm 100 is mounted on the housing 22. It could be fixedly mounted thereon. However, as will be described in more detail below, it is here mounted so as to be able to move between a retracted position (or "parked position") in which it does not interfere with the implementation of the other functions of the apparatus 10, and a position of use.

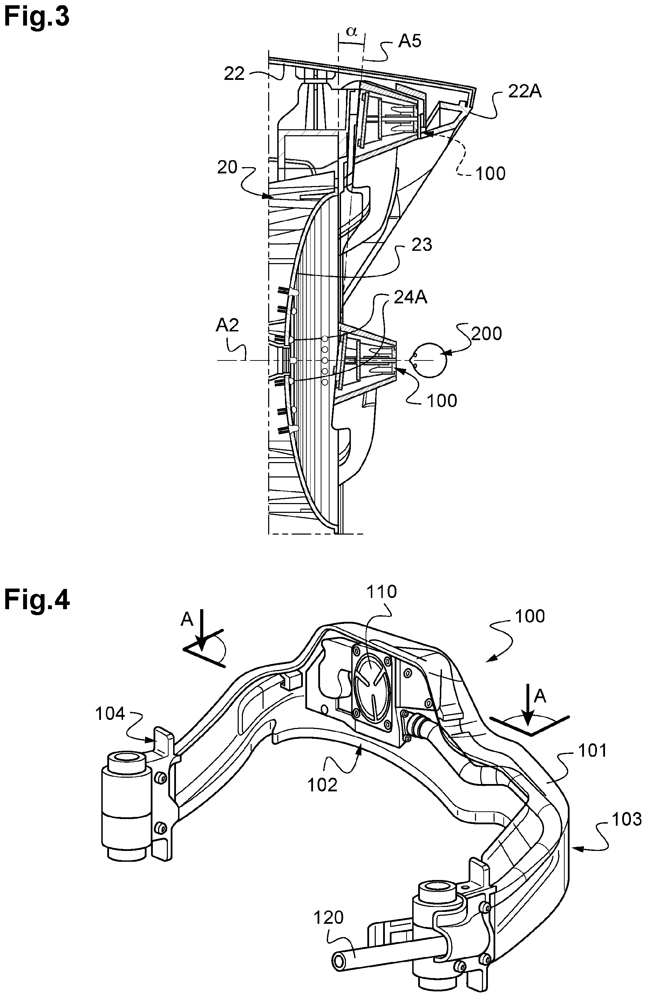

This arm 100 comprises a shell 101 illustrated in transparency in Figure 4. This shell 101 is for example formed of two parts assembled so as to delimit an interior volume between them.

The arm is curved, here in an arc of a circle, and has two ends that are mounted on the base 20.

Consequently, the shell 101 has a U shape, with a central part 102 and, on either side of the central part, two end parts 103, 104. It extends in a medium plane which passes through the eyes of the individual when the head of the latter rests on the support 25 and when the arm 100 is in position of use.

The central part 102 of the shell 101 faces forward, that is to say towards the head of the individual, while the end parts 103, 104 are folded backwards.

In the position of use, the central part 102 is thus turned towards the eye 201 of the individual on whom measurements must be carried out.

One of the end parts 103 houses an inlet pipe 120 connected to an air actuator of the air supply means 30 in order to supply air.

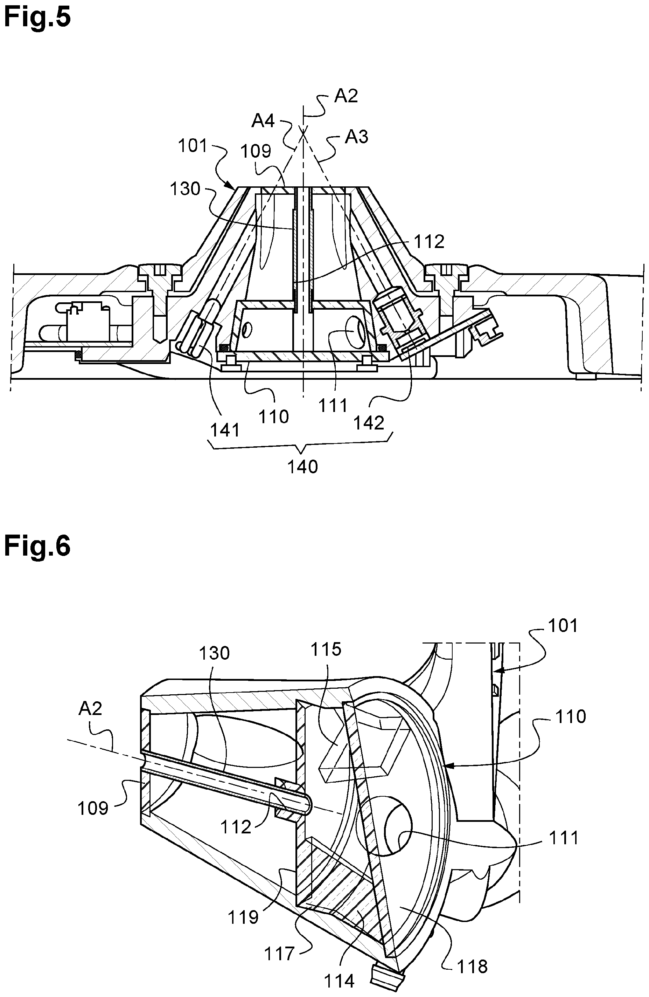

As shown in Figures 5 and 6, the central part 102 forms or houses a plenum chamber body 110.

In other words, a chamber is provided in this central part 102. This chamber is intended to receive the air pulse, to form a plenum, and to return the smoothed air towards the eye of the individual. This chamber is designed here to receive this air pulse laterally (via the inlet pipe 120 housed in the first end part 103 of the arm 100), and to eject it axially, towards the individual's eye.

In the illustrated embodiment, the plenum chamber is delimited by a body 110 distinct from the shell 101 and fixed inside the latter. But in a variant, this body could be formed by the shell 101.

We can describe this body 110 in more detail.

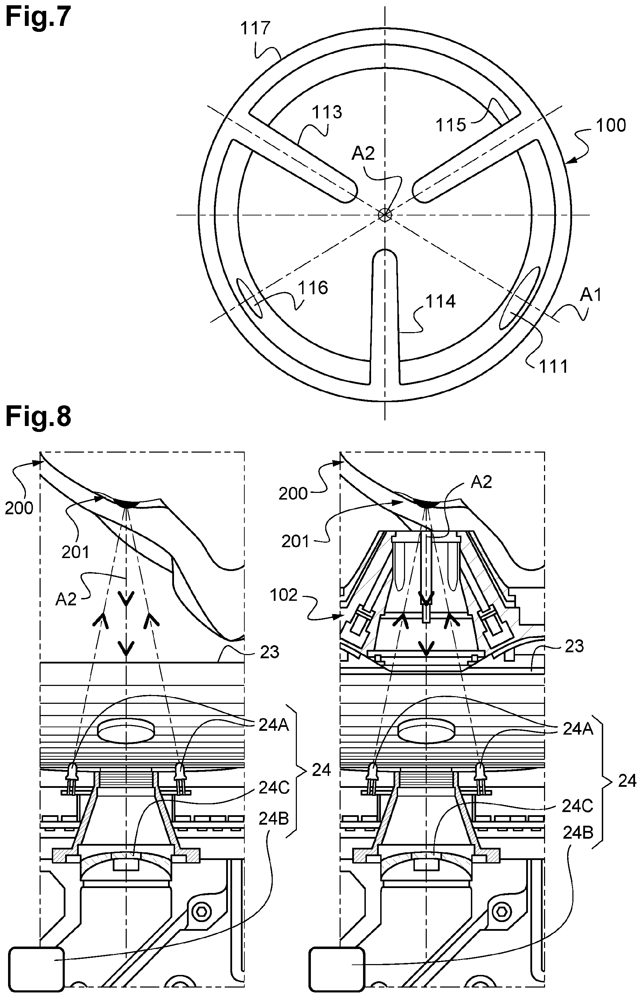

As shown in Figures 6 and 7, this plenum chamber body 110 comprises a side wall 117 and two end walls 118, 119 closing extremities of the side wall 117. It thus delimits an airtight volume (when the openings described below are closed)

In a preferred embodiment, both end walls 118, 119 are plane. They are preferably not parallel to each other. Non-parallel walls avoid light emitted by LEDs (in practice light emitting diodes used for the positioning of the apparatus in front of one of the individual eye) to be reflected toward the individual.

They are also preferably transparent to at least a range of wavelengths included in the infrared and/or visible domain.

Here, a main part of the plenum chamber body 110 is made of black anodized aluminum. But the end walls 118, 119 are made of transparent material, namely in plastic or glass. The side wall 117 could be cylindrical of revolution. However, here it is rather conical, preferably of revolution around an outlet axis A2.

This plenum chamber body 110 has a fluid inlet 111 connected to an end of the inlet pipe 120 (that is connected to the air actuator of the air supply means 30), and a fluid outlet 112 connected to an outlet pipe 130.

In a preferred embodiment, the fluid inlet 111 is located in the side wall 117 and the fluid outlet 112 is located in one of the end walls 119 (named herein after the front end wall 119).

The fluid inlet 111 has a circular shape and is here located between the end walls 118, 119.

Its diameter is here greater than half the distance between the end walls 118, 119.

The flow arrives in the chamber by this fluid inlet 111 along an inlet axis A1. In other word, this inlet axis A1 corresponds to the mean axis of the flow at its entry into the plenum chamber. We can consider that this axis coincides with the axis of the fluid inlet 111.

In practice, this inlet axis A1 is radial relative to the outlet axis A2 and is coplanar with it. In other words, even if the outlet axis A2 and the inlet axis A1 could be inclined relative to each other at an angle lying between 75 and 105 degrees, they are here exactly inclined at an angle of 90°.

The fluid outlet 112 is preferably located at the center of the front end wall 119 and is centered on the outlet axis A2.

Its diameter is lower than half the one of the fluid inlet 111.

When a pulse of air arrives from the inlet pipe 120 along the inlet axis A1, it fills the plenum chamber delimited by the body 110 and exits from this chamber by this fluid outlet 112 along the outlet axis A2.

The outlet pipe 130 has a shape of a tube of revolution about the outlet axis A2, with an end engaged into the fluid outlet 112.

To avoid turbulence as much as possible at its exit from the outlet pipe 130, the plenum chamber body 110 comprises at least one inner wall 113, 114, 115 extending substantially radially with respect to the outlet axis A2, inside the plenum chamber.

It preferably comprises at least two distinct inner walls 113, 114, 115. Here, it comprises exactly three distinct inner walls 113, 114, 115.

In a variant, it could comprise four inner walls or more, but the number of three is considered as the best mode since it is a good compromise between air smoothing and pressure losses generated on the air flow. Indeed, the greater the number of internal walls, the higher these losses will be.

Each inner wall 113, 114, 115 preferably extends from one of the end walls 118, 119 until the other one.

Moreover, each inner wall 113, 114, 115 preferably extends from the side wall 117, toward the fluid outlet 112. In practice, they have a free edge that extends at a distance from this fluid outlet 112. If their free edges bordered this fluid outlet, they would in fact be too close to each other for the air to pass without difficulty, which would generate significant pressure losses.

The inner walls 113, 114, 115 are regularly distributed around the outlet axis A2, here at 120° from each other.

Their free edges are here rectilinear and parallel to the outlet axis A2. Alternatively, they could be inclined so that the inner walls are longer on the side of the front end wall 119 than on the side of the rear end wall 118.

A first of the three distinct inner walls 113 is located opposite to the fluid inlet 111. Consequently, when it enters into the plenum chamber, the air pulse is divided symmetrically on either side of this first wall then circulates in the chamber bypassing the other inner walls 114, 115 so as to avoid turbulence as much as possible when it comes out the chamber.

As clearly shown in Figure 6, the outlet 112 provided in the front end wall 119 is bordered on its front side by a rim, which allows the outlet pipe 130 to be engaged in this opening through one of its ends. Here, this end does not enter the plenum chamber.

The outlet pipe has a ratio between length and diameter adjusted so as to obtain an air flow which is as rectilinear as possible and to guarantee the correct distance of applanation with the corneal surface to ensure the measurement.

To guarantee its fixation, this pipe is then engaged at its opposite end through an outlet plate 109 which is parallel to the front end wall 119 of the body 110 and which is also transparent. This outlet plate 109 is here fixed in the shell 101 of the central part of the arm 100.

To summarize, the inlet pipe 120, the plenum chamber body 110 and the outlet pipe 130 form together directing means suitable to eject the fluid coming from the fluid supply means 30 toward the eye 201 of the individual 200, along the outlet axis A2, when the individual's head rests on the individual's head support 25 and when the tonometer arm 100 is in position of use.

All of the elements described above therefore make it possible to generate a pulse of air on the eye of the individual whose head rests on the support 25.

Preferably, as shown in Figure 5, the arm 100 then carries optical means 140 making it possible to determine the intraocular pressure of the eye which receives the air flow.

These optical means 140 take advantage of the fact that when the eye receives this air pulse, its corneal surface undergoes a sequence of shape changes: starting from its normal convex state, flattening, becoming concave, and then returning to its normal convex state.

Consequently, during this sequence, the surface of the cornea is flat at two distinct instants.

The optical means 140 are then provided to detect at least the first of these two instants, so that the control unit can deduce therefrom the desired intraocular pressure.

To this end, the optical means 140 comprise a lighting unit 141 and a light sensor unit 142.

The lighting unit 141 comprises her a diode housed in the shell 101 and directed toward the front, along an axis A4 tilted and coplanar relative to the outlet axis A2.

The light sensor unit 142 comprises a photodiode housed in the shell 101 and directed toward the front, along an axis A3 tilted and coplanar relative to the outlet axis A2.

These axes A2, A3 and A4 intersect each other at a point that is to be located on the corneal surface of the individual.

Thus, at the moment when the surface of this cornea is flat, the light emitted by the diode is reflected by this surface towards the photodiode, which makes it possible to detect this short moment.

To prevent stray light from distorting the measurements, the diode and photodiode are housed inside the shell 101. Two cylindrical revolution ducts are provided in this shell 101. They extend along the axes A3 and A4, from the diode or the photodiode until in a volume delimited between the body 110 and the outlet plate 109. These ducts are colored in black in order to absorb this stray light. The internal face of the volume is also colored in black for the same reason. We can note that the shell can be made in a rigid black plastic.

The control unit is suitable to trigger an air pulse and to active the diode 141 in order to determine the precise moments when the cornea is flat.

In a preferred embodiment, it is also connected to a pressure gauge suitable to measure the pressure into the plenum chamber. As shown in Figure 7, an opening 116 is provided in the side wall 117 of the body 110 to allow these pressure measurements.

The air pulse generated automatically by the control unit gives the known pressure profile as a function of time in the plenum chamber. Consequently, the control unit can compare the instant when the cornea is flat with the pressure curve and calculate the intraocular pressure of the eye. Alternatively, of course, other systems can be used..

When the apparatus 10 must perform a function other than that of measuring intraocular pressure, it is necessary for the arm 100 to be able to shift so as not to form an obstacle to the implementation of this other function.

The arm 100 is therefore movably mounted on the base 20. It could be mounted so as to be able to pivot relative to it. However, here, it is slidingly mounted on the housing 22 along a sliding axis A5 (Figure 3).

For this purpose, the arm 100 comprises two slides located at the extremities of its end parts 103, 104, which can slide on two rails fixed to the housing 22. The two slides could also both be located on the same extremity of the arm.

To enable this movement, the inlet pipe 120 is connected to the air supply means 30 by a flexible hose.

Here, the sliding axis A5 being substantially vertically, the position of use is located below the retracted position.

The sliding axis could be exactly vertical. But in practice, the sliding axis A5 is inclined relative to the outlet axis A2 by an angle lying between 70 and 89 degrees, such that the retracted position is located at the front of the position of use.

In other words, when it descends towards its position of use, the arm 100 approaches the base 20.

By considering that the individual's head support 25 defines a support plane P1 for an individual's forehead which is orthogonal to the outlet axis A2 (Fig. 1), the tonometer arm 100 moves away from the support plane P1 (toward the rear i.e. toward the base 20) when it moves from its retracted position to its position of use.

Thanks to this slight inclination of the sliding axis A5, the arm 100 can be positioned as close as possible to the base 20 in the position of use, at an adequate distance from the individual's eye. The mobility of the arm 100 indeed exploits the concave shape of the dome, while avoiding collision with the upper part of the dome when the tonometer arm 100 moves in or out of its retracted position.

The sliding of the tonometer arm 100 is preferably motorized. Consequently, this arm is automatically driven toward the position of use when the tonometer function is selected, and toward the retracted position when any other function is selected.

A shown in Figures 1 and 3, in a preferred embodiment, the housing 22 forms, in an upper part of its protective cover, a visor 22A (or "cap") that is adapted to house the entire tonometer arm 100 when the latter is in retracted position. This visor 22A protects this arm from dust when it is not used. It also protects the individual from light coming from a ceiling light, which could be a nuisance for the individual and for the measures especially when functions other than tonometry are carried-out.

This visor 22A extends from on side to the other side of the protective cover, above the inverted dome 23.

Before measuring this intraocular pressure (or any other eye characteristic), it is necessary to ensure that the individual's eye 201 is well centered on the outlet axis A2.

To this end, the arm 100 could be equipped with positioning means (for example a camera allowing the operator to visualize the effective position of the eye in relation to a desired position).

However, these positioning means 24 are rather housed in the base 20, and more precisely here in the housing 22 of the base 20, on the central axis of the inverted dome 23.

The positioning means 24 and the tonometer arm 100 are designed such that the position of the individual's head resting on the support 25 can be determined whether the tonometer arm 100 is in retracted position (left drawing of Figure 2) or in position of use (right drawing of Figure 2). This is the reason why the end walls 118, 119 and the outlet plate 109 are transparent.

Consequently, these positioning means can be used to determine or check the position of the individual's eye 201 not only to carry-out the tonometer function (to measure the intraocular pressure value), but also to carry-out at least one of the other functions (to measure the value of another characteristic of the eye 201).

These positioning means could come in various forms and could operate according to different physical principles. They could typically include two stereoscopic cameras to determine the position of the eye.

But here, as shown in Figure 8, these positioning means 24 include at least one light-emitting diode 24A suitable to illuminate the individual's eye 210 directly (when the tonometer arm 100 is in retracted position) or through the end walls 118, 119 of the plenum chamber body 110 and the outlet plate 109 (when the tonometer arm 100 is in position of use).

They also include an acquisition unit such as a camera 24B adapted to acquire images of the individual's eye 201 directly (when the tonometer arm 100 is in retracted position) or through the end walls 118, 119 of the plenum chamber body 110 and the outlet plate 109 (when the tonometer arm 100 is in position of use).

In practice, the positioning means 24 include at least three light-emitting diodes 24A distributed along a circle centered on the outlet axis A2, on the dome 23. These diodes are directed toward the support 25 to be able to generate reflections on the cornea of the eye 201.

The camera 24B is located inside the housing 22. It is centered on the outlet axis A2. Consequently, the apex of the dome 23 is opened to let the light coming from the reflections enter the housing and reach the camera 24B.

As shown in Figure 8, the housing 22 can house a mirror to deviate this outlet axis A2 (specifically the light rays propagating along this axis). In this case, the camera 24B can be located on a deviated part of the outlet axis A2, for instance for compactness reasons.

Here, the positioning means 24 also include a collimating lens 24C located on the outlet axis A2, between the camera 24B and the opening on the apex of the inverted dome 23.

To summarize, the light-emitting diodes 24A and the camera 24B are positioned such that, in position of use, a light ray emitted by any of the diodes passes through the two transparent end walls 118, 119 of the plenum chamber body 110, is reflected by the individual 's eye 201 and comes back through the two transparent end walls 118, 119 until the camera 24B.

Therefore, the camera 24B is suitable to acquire data relative to the position of the illuminated individual's eye 201 relative to the housing 22, to verify that this eye 201 is well located on the outlet axis A2.

To this end, the control unit can proceed in various ways.

It can display on the display screen 23B a cross illustrating the position of the outlet axis A2 superimposed on the images acquired by the camera, so that the operator can adjust the height of the chin rest 25B and/or ask the individual to reposition himself correctly and/or adjust the position of the housing 22 relative to the base support 21 until the eye 201 is well centered on the cross.

Alternatively, the control unit can automatically adjust the height of the chin rest 25B relative to the base 20 if this chin rest 25B is motorized.

Still as a variant, the control unit can automatically adjust the height of the tonometer arm 100 relative to the base 20 if this arm is motorized.

In practice, here, the control unit is programmed to:
- acquire a new image by means of the camera 24B,
- detect on the acquired image the positions of the reflections of the light-emitting diodes 24A on the eye 201,
- if these reflections are located on a circle centered on the outlet axis A2, emitting a signal indicating that the eye 201 is well positioned, else,
- generate a command signal to modify the position of the housing 22
relative to the base support 21 in a direction such that the center of the circle moves toward the outlet axis A2, and then restart this process (by acquiring a new image).

The signal command enables the housing 22 to be placed in the right position to perform the measurements.

The signal indicating that the eye is well positioned can be transmitted to the screen 23B (so that this screen displays this information) or can be used by the control unit to automatically start a measure of a characteristic of the eye (intraocular pressure...).

The control unit could be programmed to implement this eye positioning process before each measurement of an eye characteristic.

In a preferred embodiment, it is also programmed to implement this eye positioning process during the measurement of at least one of these characteristics (here during the implementation of the tonometer function). The positioning means 24 and the tonometer arm 100 are indeed designed such that the light-emitting diodes 24A and the camera 24B can continue to illuminate the eye's cornea and to detect the reflections during the intraocular pression measurement, enabling the housing 22 to remain in the right position relative to the individual's eye during the whole process.

The present disclosure is in no way limited to the embodiment described and shown.

In particular, the plenum chamber body might not be transparent if the arm were equipped with its own positioning means.

According to another variant of the disclosure, the body could have a different shape. Typically, its side wall could not present a symmetry of revolution around the outlet axis A2. Its internal walls could also extend at a distance from the side wall.

Still as a variant, its internal walls could extend from one of the end walls, towards the other, but at a distance from the latter.

Still as a variant, the pulsed fluid could not be air, but any other suitable fluid capable of deforming the surface of the cornea of the individual's eye.

According to another variant, the tonometer arm 100 could be designed to move relative to the housing by rotation rather than by translation. This solution is not preferred because it would require the individual's head to move back from the head support 25 before moving the arm. But such a solution would be less expensive.

According to another variant, the optical means (used to measure the intraocular pressure) could be fitted in the housing 22 rather than in the tonometer arm 100.

## Claims

1. Apparatus (10) for determining a value of at least one characteristic of an eye (201) of an individual (200), comprising:
- a housing (22),
- a individual's head support (25),
- a tonometer arm (100) configured to determine the value of the at least one characteristic,
**characterized in that** the tonometer arm (100) is movable relative to the housing (22) between a position of use and a retracted position distinct from the position of use, and
**in that** the housing (22) comprises positioning means (24) suitable to determine the position of the individual's eye (201) relative to the housing (22) when the individual 's head rests on the individual's head support (25), whether the tonometer arm (100) is in retracted position or in position of use.

2. Apparatus (10) according to claim 1, comprising a determining unit (60) fixed relative to the housing (22) and suitable to determine a value of at least one other characteristic of the eye (201) when the individual's head rests on the individual's head support (25).

3. Apparatus (10) according to claim 1 or 2, comprising fluid supply means (30), and wherein the tonometer arm (100) includes directing means suitable to eject the fluid coming from the fluid supply means (30) toward the eye (201), along an outlet axis (A2), when the individual's head rests on the individual's head support (25) and when the tonometer arm (100) is in the position of use.

4. Apparatus (10) according to claim 3, wherein the directing means comprises:
- a plenum chamber body (110) that has a side wall (117) and two transparent end walls (118, 119) closing extremities of the side wall (117),
- an inlet pipe (120) connected to a fluid inlet (111) of the plenum chamber body (110) and to the air supply means (30) to supply the plenum chamber body (110) with the fluid,
- an outlet pipe (130) connected to a fluid outlet (112) of the plenum chamber body (110) and adapted to eject the fluid outside of the plenum chamber body (110) along the outlet axis (A2).

5. Apparatus (10) according to any one of claims 1 to 4, wherein the positioning means (24) comprise:
- at least one light-emitting diode (24A) suitable to illuminate the individual 's eye (201) through the tonometer arm (100) when this tonometer arm (100) is in position of use, and
- at least one acquisition unit (24B), such as a camera, suitable to acquire data relative to the position of the illuminated individual's eye relative to the housing (22), through the tonometer arm (100) when this tonometer arm (100) is in position of use.

6. Apparatus (10) according to claims 4 and 5, wherein the at least one light-emitting diode (24A) and the at least one acquisition unit (24B) are positioned such that a light ray emitted by the at least one light-emitting diode (24A) passes through the two transparent end walls (118, 119) of the plenum chamber body (110), is reflected by the individual 's eye (201) and comes back through the two transparent end walls (118, 119) until the at least one acquisition unit (24B).

7. Apparatus (10) according to claim 5 or 6, wherein the positioning means (24) comprise at least three light-emitting diodes (24A) distributed along a circle centered on an outlet axis (A2), and the acquisition unit (24B) is centered on the outlet axis (A2).

8. Apparatus (10) according to any one of claims 1 to 7, wherein the housing (22) includes a dome (23) facing the individual 's head support (25), the apex of which is directed away from the individual's head support (25).

9. Apparatus (10) according to claim 7 and 8, wherein the light-emitting diodes (24A) are located on the dome (23) and the acquisition unit (24B) is located inside the housing (22), the apex of the dome (23) being opened.

10. Apparatus (10) according to any one of claims 1 to 9, wherein the tonometer arm (100) includes directing means for ejecting a fluid toward the eye (201) of the individual (200) along an outlet axis (A2) and wherein the tonometer arm (100) is mounted sliding relative to the housing (22) along a sliding axis (A5) tilted relative to the outlet axis (A2).

11. Apparatus (10) according to claim 10, wherein the individual 's head support (25) defines a support plane (P1) for a individual 's forehead which is orthogonal to the outlet axis (A2) and wherein the sliding axis (A5) is tilted relative to the outlet axis (A2) such that the tonometer arm (100) moves away from the support plane (P1) when it moves from its retracted position to its position of use.

12. Apparatus (10) according to claim 10 or 11, wherein the sliding axis (A5) is inclined relative to the outlet axis (A2) by an angle (α) lying between 70 and 89 degrees.

13. Apparatus (10) according to any one of claims 10 to 12, wherein the sliding of the tonometer arm (100) is motorized.

14. Apparatus (10) according to any one of claims 1 to 13, wherein the value of the at least one characteristic is determined by measuring means (140), the measuring means (140) comprising a lighting unit (141) and a light sensor unit (142) that are housed in the tonometer arm (100) and oriented along axes (A3, A4) intersecting each other.

15. Apparatus (10) according to any one of claims 1 to 14, wherein the housing (22) comprises a visor (22A) that is adapted to house the tonometer arm (100) in retracted position.
